Europäisches Patentamt

European Patent Office

Office européen des brevets

(19)

(11) Veröffentlichungsnummer: **0 011 718 B1**

# EUROPÄISCHE PATENTSCHRIFT

(12)

(45) Veröffentlichungstag der Patentschrift:
01.12.82

(51) Int. Cl.³: **A 61 K 9/50**, A 61 K 35/78

(21) Anmeldenummer: 79104194.0

(22) Anmeldetag: 29.10.79

(54) Stabile Valepotriat-Zubereitungen und Verfahren zu deren Herstellung.

(30) Priorität: 11.11.78 DE 2849029

(43) Veröffentlichungstag der Anmeldung:
11.06.80 Patentblatt 80/12

(45) Bekanntmachung des Hinweises auf die Patenterteilung:
01.12.82 Patentblatt 82/48

(84) Benannte Vertragsstaaten:
AT BE CH DE FR GB IT LU NL SE

(56) Entgegenhaltungen:
DE-A-2 654 709
DE-B-1 191 515
DE-B-2 230 626
FR-A-1 266 902
FR-A-2 057 561

Moderne Arzneimittel Seite 125 Rote Liste 1975 82066 AC

Die Akte enthält technische Angaben, die nach dem Eingang der Anmeldung eingereicht wurden und die nicht in dieser Patentschrift enthalten sind.

(73) Patentinhaber: Kali-Chemie Pharma GmbH,
Hans-Böckler-Allee 20 Postfach 220,
D-3000 Hannover 1 (DE)

(72) Erfinder: Wischniewski, Martin, Dr.rer.nat., Kattowitzer Weg 24, D-3057 Neustadt a. Rbge. (DE)
Erfinder: Feicho, Lutz, Paracelsusweg 17,
D-3057 Neustadt a. Rbge. (DE)
Erfinder: Althaus, Werner, Dr.rer.nat., Liebigstrasse 9,
D-3167 Burgdorf (DE)

(74) Vertreter: Lauer, Dieter, Dr., c/o Kali-Chemie Aktiengesellschaft Postfach 220,
D-3000 Hannover 1 (DE)

Stabile Valepotriat-Zubereitungen und Verfahren zu deren Herstellung

Die Erfindung betrifft stabile Valepotriat-Zubereitungen und Verfahren zu deren Herstellung.

Unter Valepotriaten versteht man eine Reihe von therapeutisch wertvollen Stoffen, die sich aus Wurzeln und Rizomen verschiedener Valerianaceen- und Kentranthus-Arten gewinnen lassen. Die wichtigsten Vertreter der Valepotriate sind Valtratum, Isovaltratum, Didrovaltratum und Acevaltratum. Sie lassen sich aus der getrockneten Droge in Mengen bis zu etwa 5 Gew.-% extrahieren. Die Anteile der einzelnen Valepotriate untereinander unterscheiden sich je nach Provenienz der Droge, wobei Didrovaltratum in Drogen asiatischer Provenienz und Valtratum/Isovaltratum in Drogen europäischer oder mexikanischer Provenienz überwiegen, während Acevaltratum jeweils in untergeordneten Anteilen vorliegt.

Die Valepotriate sind äusserst empfindliche Substanzen und werden, obgleich in Wasser praktisch unlöslich, unter dem Einfluss von Feuchtigkeit sowie von Wärme, Licht und chemischen Agentien schnell zersetzt. Sie lassen sich daher weder in der Droge noch in isolierter Form unter normalen Lagerbedingungen ausreichend lange stabil halten.

Es hat daher nicht an Versuchen gefehlt, Valepotriate so zuzubereiten, dass sie sich über längere Zeit stabil erhalten.

So beschreibt die DE-AS 2 230 626 ein Baldrian-Präparat, das durch Lösen eines Valerianaceen-Extrakts in einem mehrwertigen Alkohol, einem fetten Öl oder einem ätherischen Öl oder Mischungen daraus hergestellt ist und das nach fünfmonatiger Lagerung bei Zimmertemperatur keine nennenswerten Abbauerscheinungen zeigt. Bei Lagerung unter 10 °C soll die beschriebene Lösung mindestens 12 Monate stabil sein. Ohne näheren Hinweis auf die Auswirkungen auf die Stabilität ist dort ausserdem die Mikroverkapselung der beschriebenen Lösung erwähnt. Die dort beobachtete 5- bis 12monatige Stabilität ist noch kein hinreichender Zeitraum für fertige Arzneiformen. Untersuchungen über einen längeren Zeitraum lassen auch bei einem derartigen Präparat Zersetzungserscheinungen beobachten.

Ausserdem schlägt die DE-OS 2 654 709 vor, Baldrianextraktpräparate durch Entwässerung auf einen Restwassergehalt von weniger als 0,5% zu stabilisieren. Dieser Vorschlag erfordert zusätzliche, zum Teil extreme Massnahmen, wie z. B. Arbeiten unter Stickstoff, und lässt, wie die dort aufgeführten, nicht über 6 Monate hinausgehenden Beobachtungen zeigen, ebenfalls nur mässige Stabilität erzielen.

Der Erfindung liegt daher die Aufgabe zugrunde, unter Vermeidung der beim Stand der Technik gegebenen Nachteile Valepotriat-Zubereitungen zur Verfügung zu stellen, die im wünschenswerten Ausmass langanhaltend stabil sind.

Die Erfindung löst diese Aufgabe ausgehend von den bekannten Valepotriat-Zubereitungen in Form von Mikrokapseln, bei denen Mikrotröpfchen, die Valerianaceen-Extrakte enthalten, von wasserlöslichen festen, physiologisch indifferenten Hüllstoffen eingeschlossen sind, dadurch, dass die Mikrotröpfchen aus auf rund 80 Gew.-% Valepotriate angereicherten, öligen bis halbfesten, vom spezifischen Geruch der Isovaleriansäure befreiten Valerianaceen-Extrakten und die Hüllstoffe aus Gummi arabicum, Methylcellulose oder Mischpolymerisaten aus Polyvinylpyrrolidon und Polyvinylacetat bestehen.

Derartige Zubereitungen zeigen über einen bis zu drei Jahre umfassenden Beobachtungszeitraum keinen Stabilitätsfall, was um so überraschender ist, als bei ihrer Herstellung keine extremen Vorsichtsmassnahmen eingehalten werden und die in Form eines schüttfähigen Pulvers aus Mikrokapseln vorliegenden Zubereitungen nach der Bestimmungsmethode nach Karl Fischer einen Wassergehalt von etwa 3% aufweisen. Die erfindungsgemässen mikroverkapselten Valepotriat-Zubereitungen überführen den flüssigen bzw. halbfesten Wirkstoff in ein trockenes Pulver, dass sich technologisch leicht in die verschiedenen galenischen Formen weiterverarbeiten lässt. Die Zubereitungen kaschieren ausserdem die sehr geruchsintensiven Valepotriate. Sie brauchen nicht wie die reinen Valepotriate oder die bekannten Lösungen unter Kühlung aufbewahrt zu werden.

Wichtig für die Stabilität ist jedoch, dass der verwendete Extrakt im wesentlichen von unspezifischen Begleitstoffen befreit ist. Es hat sich nämlich gezeigt, dass derartige Begleitstoffe, obwohl in der Droge mit den Valepotriaten vergesellschaftet, deren Stabilität beeinträchtigen.

Man erhält den für die erfindungsgemässen Zubereitungen zu verwendenden, von unspezifischen Begleitstoffen im wesentlichen befreiten Extrakt in bekannter Weise (DE-PS 1 191 515) durch Extraktion der pulverisierten Drogen mit niedrigsiedenden lipophilen Lösungsmitteln bei Temperaturen unter etwa 30 °C und Entfernen des Lösungsmittels im Vakuum bei Temperaturen von ebenfalls unter etwa 30 °C. Bei der Extraktion ist es vorteilhaft, einen schwachsauren pH aufrechtzuerhalten, was, sofern die Extraktionsmischung aufgrund saurer Begleitsubstanzen in der Droge nicht von selbst den genannten pH aufweist, durch Zugabe saurer Substanzen bewerkstelligt werden kann. Der so erhaltene Rohextrakt wird dann in 90%iger Essigsäure aufgenommen. Aus der zurückbleibenden Essigsäurephase werden dann anschliessend nach Verdünnen mit Wasser auf das 1,5- bis 2fache Volumen die Valepotriate mittels mit Wasser nicht mischbarer niedrigsiedender lipophiler Lösungsmittel ausgeschüttelt. Nach Entfernen des Lösungsmittels im Vakuum werden die Valepotriate je nach Provenienz der Droge als ölige bis halbfeste, schmalzartige, im Aussehen an teilweise kristallisierten Honig erinnernde Masse mit charakteristischem, vom spezifischen Geruch der Isovaleriansäure freien Eigengeruch erhalten. Diese Masse wird nachfolgend als «genuine Valepotriat-Fraktion» bezeichnet. Sie enthält rund 80 Gew.-% Valepotriate.

Als Hüllstoffe werden die auch sonst zur Mikroverkapselung von Arzneistoffen verwendeten Stoffe Gummi arabicum, Methylcellulose oder Mischpolymerisate aus Polyvinylpyrrolidon und Polyvinylacetat verwendet.

Zur Herstellung der erfindungsgemässen Zubereitungen werden die Hüllstoffe, gegebenenfalls unter Erwärmung, in Wasser gelöst und mit der gegebenenfalls vorher erwärmten genuinen Valepotriat-Fraktion intensiv vermischt und die Mischung zu Mikrokapseln verarbeitet. Die Mikroverkapselung kann in üblicher Weise durch Sprühtrocknung erfolgen.

Für die wässrigen Lösungen der Hüllstoffe haben sich Konzentrationen zwischen 35 Gewichtsteilen Hüllstoffe und 65 Gewichtsteilen Wasser und 2,5 Gewichtsteilen Hüllstoffe und 97,5 Gewichtsteilen Wasser bewährt. Die Grenzen ergeben sich aus dem Umstand, dass zu konzentrierte und damit zu viskose Lösungen nicht mehr sprühgetrocknet werden können, während sich zu verdünnte Lösungen nicht mehr wirtschaftlich sprühtrocknen lassen.

Das Verhältnis genuine Valepotriat-Fraktion : Hüllstoff kann zwischen 50 : 50 Gewichtsteilen und 2,5 : 97,5 Gewichtsteilen schwanken. Eine höhere Konzentration an Extrakt als 50 Gewichtsprozent (bezogen auf die fertige Mikrokapsel) ist zwar möglich, ergibt aber kein trockenes, fliessfähiges Pulver mehr, während sich die untere Grenze von 2,5 Gewichtsprozent aus Dosierungsgesichtspunkten ergibt. Bei dem erwähnten Gehalt von 80 Gewichtsprozent Valepotriaten in der genuinen Valepotriat-Fraktion entsprechen die genannten Konzentrationen einem Wirkstoffgehalt in den Mikrokapseln zwischen 40 und 2 Gew.-%.

Die nachfolgenden Beispiele erläutern die Erfindung. Die Beispiele sind dabei in tabellarischer Form zusammengefasst.

### A. Ausgangsstoffe

|  | Beispiel 1 | Beispiel 2 | Beispiel 3 |
|---|---|---|---|
| Gummi arabicum | 3,0 kg | 3,0 kg | 2,5 kg |
| in Wasser | 6,0 kg | 6,0 kg | 5,0 kg |
| Methylcellulose 25 cps | 2,0 kg | 2,0 kg | – |
| in Wasser | 28,0 kg | 22,0 kg | – |
| Luviskol VA64* | – | 1,0 kg | 5,0 kg |
| in Wasser | – | 5,0 kg | 18,0 kg |
| genuine Valepotriat-Fraktion | 5,0 kg | 5,0 kg | 2,5 kg |

\* Mischpolymerisat aus 6 Teilen Polyvinylpyrrolidon und 4 Teilen Polyvinylacetat, erhältlich von der Fa. BASF.

### B. Herstellung

Die wässrigen Lösungen von Gummi arabicum, Methylcellulose und Luviskol werden gemischt und auf 40°C erwärmt. Die ebenfalls auf 40°C erwärmte, geschmolzene genuine Valepotriat-Fraktion wird unter intensivem Rühren der Lösung der Hüllstoffe zugesetzt. Im Fall des Beispiels 3 werden dieser Lösung weitere 18,0 kg Wasser zugefügt. Die Mischung wird mittels einer Zahnkolloidmühle (Puc-Viskosator) homogenisiert. Die erhaltene Emulsion wird anschliessend sofort auf einem Zentrifugal-Zerstäubungstrockner (Krause-Turm) sprühgetrocknet.

### C. Stabilität

Die Bestimmung der Valepotriate erfolgte mit Hilfe der sog. Epoxid-Methode. Dabei werden etwa 200 mg Substanz in ein hohes 50-ml-Becherglas genau eingewogen und mit 500 mg Tetraethylammoniumjodid versetzt. Nach Zugabe von 30 ml eines Gemisches aus 50 Teilen Chloroform, 30 Teilen Eisessig und 20 Teilen Essigsäureanhydrid wird auf einem elektrisch beheizbaren Magnetrührer in einem Grafitbad unter Rühren 25 Min. auf 65°C erhitzt. Das Bad soll bereits beim Aufstellen des Becherglases die vorgeschriebene Temperatur haben. Das Becherglas wird mit einem Uhrglas bedeckt.

Nach genau 25 Minuten wird das Reaktionsgemisch noch warm mit 0,1 N Perchlorsäure mittels eines Metrohm-Potentiographen titriert. Dabei wird eine kombinierte Glaselektrode, die auf den Bereich pH 14 eingestellt ist, verwendet.

Die Berechnung des Valepotriatgehaltes erfolgt gemäss nachstehender Formel:

$$\frac{\text{verbr. ml } HClO_4 \times 42,5 \times 100}{\text{Einwaage in mg}} = \% \text{ Valepotriate}$$

Die Ergebnisse sind folgende:

| Zeitpunkt der Bestimmung | Beispiel 1 | Beispiel 2 | Beispiel 3 |
|---|---|---|---|
|  | Gew.-% Valepotriate | | |
| unmittelbar nach Herstellung | 39,9 | 38,0 | 20,15 |
| 11 Monate später | 39,9 | 38,9 | – |
| 36 Monate später | – | – | 22,0 |

## Patentansprüche

1. Valepotriat-Zubereitungen in Form von Mikrokapseln, bei denen Mikrotröpfchen, die Valerianaceen-Extrakte enthalten, von wasserlöslichen festen, physiologisch indifferenten Hüllstoffen eingeschlossen sind, dadurch gekennzeichnet, dass die Mikrotröpfchen aus auf rund 80 Gew.-% Valepotriate angereicherten, öligen bis halbfesten, vom spezifischen Geruch der Isovaleriansäure befreiten Valerianaceen-Extrakten und die Hüllstoffe aus Gummi arabicum, Methylcellulose oder Mischpolymerisaten aus Polyvinylpyrrolidon und Polyvinylacetat bestehen.

2. Zubereitungen nach Anspruch 1, dadurch gekennzeichnet, dass sie 2,5 bis 50 Gew.-% Extrakt enthalten.

3. Verfahren zur Herstellung von Valepotriat-Zubereitungen in Form von Mikrokapseln, bei denen Mikrotröpfchen, die Valerianaceen-Extrakte enthalten, von wasserlöslichen festen, physiologisch indifferenten Hüllstoffen eingeschlossen sind, dadurch gekennzeichnet, dass man auf rund 80 Gew.-% Valepotriate angereicherte, ölige bis halbfeste, vom spezifischen Geruch der Isovaleriansäure befreite Valerianaceen-Extrakte mit einer wässrigen Lösung aus Gummi arabicum, Methylcellulose oder Mischpolymerisaten aus Polyvinylpyrrolidon und Polyvinylacetat vermischt und die Mischung zu Mikrokapseln verarbeitet.

4. Verfahren nach Anspruch 3, dadurch gekennzeichnet, dass die wässrige Lösung 2,5 bis 35 Gew.-% Hüllstoffe enthält.

5. Verfahren nach Anspruch 3 oder 4, dadurch gekennzeichnet, dass die Mischung 2,5 bis 50 Gew.-% Extrakt, bezogen auf die festen Hüllstoffe, enthält.

6. Verfahren nach einem der Ansprüche 3 bis 5, dadurch gekennzeichnet, dass die Mikroverkapselung durch Sprühtrocknen der Mischung erfolgt.

## Claims

1. Valepotriate preparations in the form of microcapsules, in which micro-droplets, which contain extracts of Valerianaceae are enclosed by water-soluble, solid, physiologically indifferent covering materials, characterised in that the micro-droplets consist of extracts of Valerianaceae which have been concentrated to approximately 80% by weight of valepotriates, which are oleaginous to semi-solid, and which are free of the specific odour of isovalerianic acid, and the covering materials consist of gum arabic, methyl cellulose or copolymers of polyvinyl pyrrolidone and polyvinyl acetate.

2. Preparations according to claim 1 characterised in that they contain 2.5 to 50% by weight of extract.

3. Method for the production of valepotriate preparations in the form of microcapsules, in which microdroplets, which contain extracts of Valerianaceae, are enclosed by water-soluble, solid, physiologically indifferent covering materials, characterised in that extracts of Valerianaceae which have been concentrated to approximately 80% by weight valepotriates, which are oleaginous to semi-solid, and which are freed of the specific odour of isovalerianic acid, are mixed with an aqueous solution of gum arabic, methyl cellulose or copolymers of polyvinyl pyrrolidone and polyvinyl acetate and the mixture is processed into microcapsules.

4. Method according to claim 3, characterised in that the aqueous solution contains 2.5 to 35% by weight covering materials.

5. Method according to claim 3 or 4, characterised in that the mixture contains 2.5 to 50% by weight of extract based on the solid covering substances.

6. Method according to one of claims 3 to 5 characterised in that the microencapsulation takes place by spray drying of the mixture.

## Revendications

1. Préparations de valépotriates sous forme de microcapsules dans lesquelles des microgouttelettes contenant des extraits de valérianacées sont enfermées dans des substances d'enrobage solides, hydrosolubles et physiologiquement indifférentes, caractérisées en ce que les microgouttelettes sont constituées d'extraits de valérianacées huileux à semi-solides, enrichis à environ 80% en poids de valépotriates et débarassés de l'odeur spécifique de l'acide isovalérianique, tandis que les substances d'enrobage sont constituées de gomme arabique, de méthyl-cellulose ou de copolymères de polyvinylpyrrolidone et d'acétate de polyvinyle.

2. Préparations suivant la revendication 1, caractérisées en ce qu'elles contiennent 2,5 à 50% en poids d'extrait.

3. Procédé en vue d'obtenir des préparations de valépotriates sous forme de microcapsules dans lesquelles des microgouttelettes contenant des extraits de valérianacées sont enfermées dans des substances d'enrobage solides, hydrosolubles et physiologiquement indifférentes, caractérisé en ce qu'on mélange des extraits de valérianacées huileux à semi-solides, enrichis à environ 80% de valépotriates et débarrassés de l'odeur spécifique de l'acide isovalérianique avec une solution aqueuse de gomme arabique, de méthyl-cellulose ou de copolymères de polyvinylpyrrolidone et d'acétate de polyvinyle, puis on transforme le mélange en microcapsules.

4. Procédé suivant la revendication 3, caractérisé en ce que la solution aqueuse contient 2,5 à 35% en poids de substances d'enrobage.

5. Procédé suivant la revendication 3 ou 4, caractérisé en ce que le mélange contient 2,5 à 50% en poids d'extrait, calculé sur les substances d'enrobage solides.

6. Procédé suivant une des revendications 3 à 5, caractérisé en ce que le micro-encapsulage a lieu en soumettant le mélange à un séchage par pulvérisation.